# EUROPEAN PATENT APPLICATION

(11) **EP 3 604 460 A1**
(43) Date of publication of application: **05.02.2020**
(21) Application number: 18777950.9
(22) Date of filing: 19.03.2018
(51) Int. Cl.: C09D 11/30, A61K 8/81, A61Q 1/00, B41J 2/01, B41M 5/00

(54) **INK COMPOSITION FOR INK-JET PRINTER**

(30) Priority: 29.03.2017 JP 2017065176
(71) Applicant: Shiseido Company, Ltd., Tokyo 104-0061 (JP)
(72) Inventor: URASHIMA, Toshifumi, Kakegawa-shi Shizuoka 436-0047 (JP); Aso, Daisuke, Kakegawa-shi Shizuoka 436-0047 (JP); YOSHINO, Nobuyuki, Kakegawa-shi Shizuoka 436-0047 (JP); HIRAYAMA, Takehiro, Yokohama-shi Kanagawa 224-8558 (JP)
(74) Representative: Ter Meer Steinmeister & Partner
(86) International application number: PCT/JP2018/010896
(87) International publication number: WO 2018/180732

(57) **Abstract**

The purpose of the present invention is to provide an ink composition for ink-jet printers which is for painting or drawing on cosmetic surfaces, etc. using an ink-jet printer and which is excellent in terms of the dispersibility and redispersibility of a pigment, etc. and consists only of components that are permitted to be incorporated into cosmetics. The present invention relates to an ink composition for ink-jet printers, characterized by containing at least one copolymer selected from between (a) an alkyl acrylate/diacetone acrylamide copolymer and (b) an acrylate/C₁₋₁₈-alkyl acrylate/C₁₋₈-alkylacrylamide copolymer.

## Description

### Technical Field

The present invention relates to an ink composition for an ink-jet (inkjet) printer. More particularly, the present invention relates to the ink composition (formulation) with which an ink-jet printer enables a pattern (design), including a fine letter and a graphic (figure), on a cosmetic surface to print linearly, and the ink composition is excellent in dispersibility (or re-dispersibility) of the blended powder such as pigment and hardly causing clogging at the printer (nozzle) head.

### Background Art

Decoration on the surface of a solid cosmetic such as a foundation, a face powder, an eye shadow and/or a blusher has been performed by printing the pattern including a variety of letters and graphics.

In Patent Document 1, decoration is provided by propelling a decorative agent, preferably having a color different from the color of the cosmetic per se, onto the surface of the solid cosmetic per se such as a foundation, to form a decorative layer followed by irradiating and processing the decorative layer with laser light. However, according to this method, only two colors, i.e., the cosmetic per se and the decorative layer, are expressible despite requiring an expensive laser processing apparatus to carry out.

Providing decoration using a printing technique is also considered, and in fact, for example, a stamp printing or a pad printing (e.g., Tampo Print®) is applied, but the sharpness is unsatisfied and it is difficult to print a more delicate line finer than 200 µm. Patent Document 2 describes a method of printing a drawing on a cosmetic surface using an electrostatic printing technique for which the screen with the printing pattern is mandatory, so that there is a problem that the edge portions of the printed subject are blurred.

Patent Document 3 discloses a cosmetic of which surface is printed by the ink-jet printer, wherein the surface of the cosmetic is obtained with a white ink layer followed by printing a drawing in a color ink on (over) the white ink layer. Whereas, a conventional ink-jet printer ink has a problem in which when the dispersibility and/or re-dispersibility of the blended powder component such as pigment is poor, clogging at a nozzle of the ink-jet printer takes place.

### Citation List

### Patent Literature

Patent Document 1: JP-A 2006-62980
Patent Document 2: JP-A 2005-144935
Patent Document 3: JP-A 2016-216416

### Summary of Invention

### Technical Problem

The present invention has been made in view of the drawbacks of the conventional art, and an object of the present invention is to provide an ink composition to print a drawing on a cosmetic surface or the like with an ink-jet printer, wherein the ink composition for the ink-jet printer is excellent in the dispersibility and re-dispersibility of such as pigment and comprises only components approved to formulate into cosmetics.

### Solution to Problem

The present inventors have studied diligently and found that an ink composition is excellent in the dispersibility and re-dispersibility of a powder and hardly cause clogging at a nozzle by selecting a particular substance as the dispersing agent blended in the ink, thus completing the present invention.

Specifically, the present invention provides an ink composition for ink-jet printer, comprising at least one copolymer selected from a group consisting of (a) (alkyl acrylate/diacetone acrylamide) copolymer and (b) (acrylate/alkyl (C1-18) acrylate/alkyl (C1-C8) acrylamide) copolymer.

### Advantageous Effects of the Invention

The ink composition (formulation) according to the present invention comprises an anionic acrylic resin specified above, so that the ink composition is excellent in the dispersibility and re-dispersibility of a powder component such as a pigment, such as aggregation of such a powder component hardly occurs resulting in that clogging at the nozzle of an ink-jet printer least likely occurs. In addition, the ink composition enables the surface of a cosmetic or a cosmetic base material to print using an ink-jet printer so that a delicate line of which width is about 100 µm or less can be sharply printed.

In some cases, dispersibility represents easiness of dispersion of a powder during the production of a product and re-dispersibility represents easiness of dispersion of the powder precipitated due to standing after the production, but herein (re)-dispersibility may be interpreted to represent and include both "dispersibility" and "re-dispersibility" without distinction unless otherwise further specified in the present specification.

### Brief Description of Drawings

FIG. 1 is a photograph showing one example of a solid cosmetic of which surface has a drawing printed using an ink-jet printer with the ink composition of the present invention.
FIG. 2 is a photograph showing other examples of solid cosmetic of which each surface has a drawing printed using an ink-jet printer with the ink composition of the present invention.
FIG. 3 is photograph showing examples of artificial nails of which each surface has a drawing printed using an ink-jet printer with the ink composition of the present invention.

### Description of Embodiments

The ink composition of the present invention comprises mandatorily at least one copolymer selected from a group consisting of (a) (alkyl acrylate/diacetone acrylamide) copolymer (INCI Name; hereinafter also referred to as "component (a)") and (b) (acrylate/alkyl (C1-18) acrylate/alkyl (C1-C8) acrylamide) copolymer (INCI Name; hereinafter also referred to as "component (b)").

Such polymers, (a) (alkyl acrylate/diacetone acrylamide) copolymer and (b) (acrylate/alkyl (C1-18) acrylate/alkyl (C1-C8) acrylamide) copolymer, are anionic acrylic polymers, and each polymer preferably blended in the form of a salt of aminomethyl propanol (AMP) in the ink composition of the present invention.

The commercially available (a) (alkyl acrylate/diacetone acrylamide) copolymer AMP may be used in the present invention, and examples thereof include Plascize® L-6330 (manufactured by GOO Chemical Co., Ltd.). Similarly, an example of the commercial products of (b) (acrylate/alkyl (C1-18) acrylate/alkyl (C1-C8) acrylamide) copolymer AMP includes Plascize® L-9909 (manufactured by GOO Chemical Co., Ltd.). Such components are capable of being formulated in a variety of cosmetics complying with the "Acrylic Resin Alkanolamine Solution" in the Japanese Standards of Quasi-Drug Ingredients (JSQI) 2006.

It is considered that the component (a) and the component (b) in the present invention act as dispersing agents in the ink composition. Whereas polyether-modified silicones, polyglycerin-modified silicones, polyether acrylic-modified silicones, polyglycerin acrylic-modified silicones, acrylic silicones, polycarboxylic acids, polycarboxylates, acrylic acid copolymers, substituted acrylic acid copolymers, polyacrylic acid, polyacrylic acid salts, and the like have been conventionally used as the dispersing agent in an ink for a ink-jet printer (see, for example, paragraph [0018] of Patent Document 3), the present inventors have first time ever found that when at least one of the component (a) and the component (b) and preferably both are blended (formulated) in the ink composition as the dispersing agent, the (re)-dispersibility of the powder component improves significantly, and the present invention is completed, accordingly.

A total amount of the component (a) and the component (b) blended in the ink composition of the present invention is 0.02 to 10% by mass, preferably 0.1 to 5% by mass, and more preferably 0.2 to 3% by mass.

As to each amount of the blended components, the amount of component (a) is 0.01 to 5% by mass, preferably 0.05 to 2.5 % by mass, and more preferably 0.1 to 1.5% by mass, and the amount of component (b) blended is 0.01 to 5% by mass, preferably 0.05 to 2.5 % by mass, and more preferably 0.1 to 1.5% by mass.

Whereas even when the ink composition of the present invention comprises either one of component (a) or component (b), it is preferred that when both component (a) and component (b) are blended together in combination, good (re)-dispersibility is achieved and the drying of the ink per se is suppressed to further improve the effect of preventing clogging at a printer head.

In addition to the component (a) and the component (b), other dispersing agents may be blended, and the total formulation (blending) amount of the component (a), the component (b) and the other dispersing agents is preferably within the above specified range (0.02 to 10% by mass, preferably 0.1 to 5% by mass, and more preferably 0.2 to 3% by mass).

The ink composition of the present invention may comprise the other components, usually blended in the ink for the ink-jet printer, than the component (a) and the component (b). For example, the following components can be listed. However, all components are preferably selected from components formulable in cosmetics.

### <Color Material>

Examples of the color material blended into the ink composition of the present invention include inorganic pigments such as white pigments, color pigments, and extender pigments, organic synthetic coloring matter such as dyes, lakes, and organic pigments, and natural coloring matter, pearlescent pigments, and functional pigments.

The inorganic pigments include white pigments such as titanium dioxide and zinc oxide, color pigments such as red oxide, yellow iron oxide, black iron oxide, ultramarine blue, Prussian blue, manganese violet, and carbon black, and extender pigments such as mica, sericite, talc, kaolin, and silicic anhydride.

The organic synthetic coloring material includes dyes such as azo dyes, xanthene dyes, quinoline dyes, triphenylmethane dyes, anthraquinone dyes, indigo dyes, nitro dyes, pyrene dyes, and nitroso dyes, lake pigments such as Red Nos. 202, 204, 206, 207, 208, and 220, dye lakes such as Yellow No. 5 and Red No. 230, and organic pigments such as azo pigments, indigo pigments, and phthalocyanine pigments.

The natural coloring material includes carotenoids, flavonoids, flavins, quinones, porphyrins, diketones, and betacyanidin coloring materials. A typical example of the pearlescent pigments is mica titanium.

Examples of the functional pigments include photochromic pigments, boron nitrides, synthetic fluorophlogopite (synthetic mica), and composite fine particle powders.

In the ink composition of the present invention, it is preferable to blend an inorganic pigment as the color material. For a white pigment, titanium dioxide is preferably used, and for a color pigment, iron oxide is preferably used.

The amount of the inorganic pigment blended in the ink composition of the present invention is 1 to 30% by mass, preferably 2 to 20% by mass, and more preferably 5 to 20% by mass.

### <Solvent>

As the solvent in the ink composition of the present invention, water or an aqueous medium is preferred. The aqueous medium means a mixture of water and a water-soluble solvent.

Examples of the water-soluble solvent include lower alcohols (having 6 or less carbon atoms) such as ethanol, polyhydric alcohols such as glycerin, diglycerin, ethylene glycol, diethylene glycol, propylene glycol, dipropylene glycol, isoprene glycol, butylene glycol, pentyl glycol, hexylene glycol, and caprylyl glycol, and sugar alcohols such as sorbitol, mannitol and erythritol.

One of the water-soluble solvents can be blended, or two or more of the water-soluble solvents can be blended in combination. Especially, blending of 1 to 20% by mass of ethanol and 5 to 60% by mass of a polyhydric alcohol is preferable in combination.

### <Surfactant>

By blending a surfactant into the ink composition of the present invention, the stability can be further improved.

The surfactant that can be used in the present invention is not particularly limited and is appropriately selected from the below listed surfactants that can be blended into cosmetics. Examples of the surfactant include polyoxyethylene type, polyhydric alcohol ester type, and ethylene oxide-propylene oxide block copolymer type nonionic surfactants and anionic, cationic, or amphoteric ionic surfactants. Specific examples include polyoxyethylene isostearyl ether, polyoxyethylene behenyl ether, polyoxyethylene glycerin monostearate, polyoxyethylene-polyoxypropylene decyl ether, sorbitan stearate, sorbitan isostearate, sorbitan sesquioleate, and sorbitan sesquiisostearate and particularly preferably include polyoxyethylene sorbitan monooleate.

The amount of the surfactant blended in the ink composition of the present invention can be, for example, 0.01 to 5% by mass, preferably 0.02 to 2% by mass, and more preferably 0.05 to 1% by mass.

In addition to the above-described components, other components usually blended in ink compositions for ink-jet printers may be blended into the ink composition of the present invention in a range of which the effects of the present invention would not be hindered. Examples of the other components can include aqueous thickening agents such as water-soluble polymers, preservatives and antioxidants, but not limited thereto.

The ink composition of the present invention can be produced according to a method usually used to produce an ink for an ink-jet printer.

Printing of fine lines and drawings on an uneven surface or a curved surface had been conventionally arduous, but such a printing is now feasible by filling an ink tank of an ink-jet printer with the ink composition of the present invention produced as described above, circulating the ink composition and printing. The nozzle diameter of the ink-jet printer is not particularly limited, but, for example, such a printer having a nozzle diameter of 20 to 30 µm is preferably used.

Typical examples of the target object on which a drawing is printed using the ink composition of the present invention include solid cosmetics such as foundations, eye shadows, lipsticks, blushers, face powders, eyebrow pencils, but the target object is not limited thereto and may be a cosmetic implement such as an artificial nail. When printing a drawing is carried out using an ink-jet printer with the ink composition of the present invention, a pattern, letters, and the like that are delicate and excellent in design properties can be printed even on an uneven surface or a curved surface.

### Examples

The present invention will be described in more detail below by giving Examples, but the present invention is not limited to these Examples. The amounts blended are expressed in % by mass unless otherwise noted.

Ink compositions were prepared with the formulation shown in the following Tables 1 and 2. Their dispersibility and re-dispersibility, and clogging at a nozzle head when printing was performed using an ink-jet printer filled with each ink composition were evaluated according to the following methods and criteria. The results are shown together in Tables 1 and 2.

### <Evaluation Methods>

The evaluation of dispersibility and re-dispersibility was carried out by observing each composition under a microscope while confirming the presence or absence of aggregates, and the like.

The evaluation of clogging at the (nozzle) head was carried out by confirming whether the presence or absence of dropouts and patchiness and the like following filling the ink-jet printer with each composition and then printing actually an image for the test printing.

### <Evaluation Criteria>

5: Very good
4: Somewhat good
3: Average (the lower limit of the acceptable level)
2: Somewhat poor
1: Poor

**[Table 1]**

| Classification | Raw material name | Comparative Example 1 | Comparative Example 2 | Comparative Example 3 | Comparative Example 4 | Comparative Example 5 | Comparative Example 6 |
|---|---|---|---|---|---|---|---|
| Pigment | Titanium dioxide or iron oxide | 10 | 10 | 10 | 10 | 10 | 10 |
| Solvent | Ethanol | 3 | 3 | 3 | 3 | 3 | 3 |
| | Glycerin | 14 | 14 | 14 | 14 | 14 | 14 |
| | Propylene glycol | 27 | 27 | 27 | 27 | 27 | 27 |
| | Ion-exchanged water | 45.8 | 45 | 45 | 45 | 45 | 45 |
| Surfactant | Polyoxyethylen e sorbitan monooleate | 0.2 | 0.2 | 0.2 | 0.2 | 0.2 | 0.2 |
| Dispersin g agent | Alkyl acrylate copolymer ammonium (*1) | - | 0.8 | - | - | - | - |
| | (Acrylate/ethylh exyl acrylate) copolymer (*2) | - | - | 0.8 | - | - | - |
| | (Acrylate/alkyl (C10-30) acrylate) crosspolymer (*3) | - | - | - | 0.8 | - | - |
| | Carbomer (*4) | - | - | - | - | 0.8 | - |
| | Acrylate copolymer (*5) | - | - | - | - | - | 0.8 |
| | Total | 100 | 100 | 100 | 100 | 100 | 100 |
| | Dispersibility | 1 | 1 | 1 | 1 | 1 | 1 |
| | Re-dispersibility | 1 | 1 | 1 | 1 | 1 | 1 |
| | Clogging of (nozzle) head | 1 | 1 | 1 | 1 | 1 | 1 |

| | | | | | | | |
|---|---|---|---|---|---|---|---|
| (*1) YODOSOL GH34F (*2) Dash Coat CG-1 (*3) PEMULEN® TR-1, 2 (*4) Carbopol® 980, 981 (*5) Carbopol® Aqua SF-1 | | | | | | | |

**[Table 2]**

| Classificatio n | Raw material name | Exampl e 1 | Exampl e 2 | Exampl e 3 | Exampl e 4 | Exampl e 5 |
|---|---|---|---|---|---|---|
| Pigment | Titanium dioxide or iron oxide | 10 | 10 | 10 | 10 | 10 |
| Solvent | Ethanol | 3 | 3 | 3 | 3 | 3 |
| | Glycerin | 14 | 14 | 14 | 14 | 14 |
| | Propylene glycol | 27 | 27 | 27 | 27 | 27 |
| | Ion-exchanged water | 45 | 45 | 45 | 45.3 | 43.8 |
| Surfactant | Polyoxyethylene sorbitan monooleate | 0.2 | 0.2 | 0.2 | 0.2 | 0.2 |
| Dispersing agent | (Alkyl acrylate/diacetone acrylamide) copolymer AMP (*6) | 0.8 | - | 0.6 | 0.4 | 1.5 |
| | (Acrylate/alkyl (C1-18) acrylate/alkyl (C1-8) acrylamide) copolymer AMP (*7) | - | 0.8 | 0.2 | 0.1 | 0.5 |
| | Total | 100 | 100 | 100 | 100 | 100 |
| | Dispersibility | 4 | 4 | 5 | 4 | 5 |
| | Re-dispersibility | 4 | 4 | 5 | 4 | 5 |
| | Clogging of head | 4 | 3 | 5 | 4 | 5 |

| | | | | | | |
|---|---|---|---|---|---|---|
| (*6) Plascize® L-6330U (*7) Plascize® L-9909B | | | | | | |

As is clear from the results shown in the above Tables 1 to 2, for all of the white inks in which titanium dioxide was blended as a pigment and the color inks in which iron oxide was blended, Examples 1 and 2, in which component (a) or component (b) was blended as a dispersing agent, were better in (re)-dispersibility than Comparative Examples 1 to 6, in which other dispersing agents were blended, and did not cause clogging at the (nozzle) head. In addition, Examples 3 to 5, in which both component (a) and component (b) were blended, was deemed the trend in which that such properties improve further.

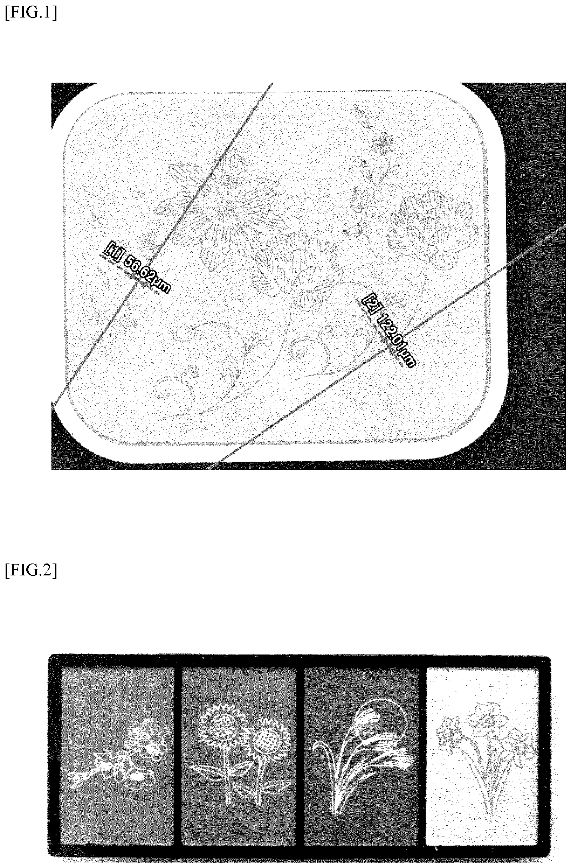

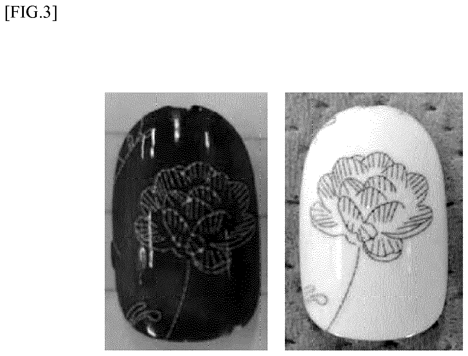

Examples of drawings printed on various cosmetics using an ink-jet printer (nozzle diameter: 20 to 30 µm) with the ink composition of the present invention are shown in FIGs.. FIG. 1 and FIG. 2 are examples in which drawings are printed on the surfaces of solid cosmetics, and it was confirmed that a precise pattern (design graphic) including fine lines of about 50 µm was printed with the ink composition of the present invention. FIG. 3 shows examples of drawings printed on each surface of artificial nails. When the ink composition of the present invention is used, such ink composition enables a delicate drawing to print even on a curved surface and an uneven surface of such as an artificial nail.

## Claims

1. An ink composition for an ink-jet printer, comprising:
at least one copolymer selected from a group consisting of (a) an (alkyl acrylate/diacetone acrylamide) copolymer and (b) an (acrylate/alkyl (C1-18) acrylate/alkyl (C1-C8) acrylamide) copolymer.

2. The ink composition according to claim 1, comprising:
(a) (alkyl acrylate/diacetone acrylamide) copolymer; and
(b) (acrylate/alkyl (C1-18) acrylate/alkyl (C1-C8) acrylamide) copolymer.

3. The ink composition according to claim 1 or 2, wherein:
said (a) (alkyl acrylate/diacetone acrylamide) copolymer and said (b) (acrylate/alkyl (C1-18) acrylate/alkyl (C1-C8) acrylamide) copolymer are salts of aminomethyl propanol (AMP).

4. The ink composition according to any one of claim 1 to 3, wherein:
a content of said (a) (alkyl acrylate/diacetone acrylamide) copolymer is in a range of 0.01 to 5% by mass of said ink composition and a content of said (b) (acrylate/alkyl (C1-18) acrylate/alkyl (C1-C8) acrylamide) copolymer is in a range of 0.01 to 5% by mass of said ink composition, and
said ink composition further comprising:
1 to 30% by mass of an inorganic pigment;
1 to 20% by mass of ethanol;
5 to 60% by mass of a polyhydric alcohol;
0.01 to 5% by mass of a surfactant; and
water.
